(19) Europäisches Patentamt
European Patent Office
Office européen des brevets

(11) **EP 4 625 310 A1**

(12) **EUROPEAN PATENT APPLICATION**
published in accordance with Art. 153(4) EPC

(43) Date of publication:
01.10.2025 Bulletin 2025/40

(51) International Patent Classification (IPC):
*G06T 7/00* (2017.01)

(21) Application number: 22966494.1

(52) Cooperative Patent Classification (CPC):
G16H 10/60; G06T 7/00; G06V 10/764;
G06V 10/7715; G06V 10/774; G06V 10/82;
G06V 20/69; G16H 30/40; G16H 50/20;
G06V 2201/03

(22) Date of filing: 24.11.2022

(86) International application number:
PCT/JP2022/043350

(87) International publication number:
WO 2024/111084 (30.05.2024 Gazette 2024/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: NEC Corporation
108-8001 Tokyo (JP)

(72) Inventor: KIYUNA, Tomoharu
Tokyo 108-8001 (JP)

(74) Representative: Betten & Resch
Patent- und Rechtsanwälte PartGmbB
Maximiliansplatz 14
80333 München (DE)

(54) **TRAINING DEVICE, PREDICTION DEVICE, TRAINING METHOD, AND RECORDING MEDIUM**

(57) In the training device, the partial image generation means generates partial images smaller than an input image from the input image. The feature space generation means generates a feature space to which feature values of the partial images are mapped, for each input image. The training data generation means generates training data by acquiring training partial images from the partial images based on the feature space. The training means trains a prediction model for predicting a probability that a predetermined feature is included in the training partial image using the training data. The prediction means performs prediction for the partial images included in the input image using a trained prediction model. Further, the training data generation means acquires training partial images used as training data in a next training, based on predicted values for the partial images in the feature space. Thus, the training of the prediction model by the training means is repeated while the training partial images are updated.

FIG. 3

100

# Description

## TECHNICAL FIELD

The present disclosure relates to a technique for predicting feature portions included in an image.

## BACKGROUND ART

There are known techniques to classify and predict images by deep learning using a neural network. In so-called supervised training, a model is trained using training data in which labels are given to input images. Patent Document 1 describes a technique of tiling the digital images of the biological sample into a group of image patches and classifying the images by applying a classifier.

## PRECEDING TECHNICAL REFERENCES

## PATENT DOCUMENT

Patent Document 1: Japanese patent application laid-open under No. 2020-533725

## SUMMARY

## PROBLEM TO BE SOLVED

When classification and prediction of images are carried out using deep learning, if the image size is large and the effective area in the image used for training small, the probability of effective data being sampled as the training data becomes small, and the training becomes inefficient. Further, if the image size is large and most of the image is effective for the training, a large number of similar data are sampled as the training data. Therefore, the diversity of the training data may become low, and the accuracy of the model obtained by the training may be lowered.

One object of the present disclosure is to perform training of a model with high accuracy while selecting effective areas as training data in a situation in which detailed labels are not given for the whole image.

## MEANS FOR SOLVING THE PROBLEM

According to an example aspect of the present invention, there is provided a training device comprising:

a partial image generation means configured to generate a plurality of partial images smaller than an input image from the input image;
a feature space generation means configured to generate a feature space to which feature values of the plurality of partial images are mapped, for each input image;
a training data generation means configured to generate training data by acquiring a plurality of training partial images from the plurality of partial images based on the feature space;
a training means configured to train a prediction model for predicting a probability that a predetermined feature is included in the training partial image using the training data; and
a prediction means configured to perform prediction for all or a part of the partial images included in the input image using a trained prediction model,
wherein the training data generation means acquires a plurality of training partial images that are used as training data in a next training, based on predicted values for all or a part of the partial images in the feature space.

According to another example aspect of the present invention, there is provided a training method executed by a computer, comprising:

generating a plurality of partial images smaller than an input image from the input image;
generating a feature space on which feature values of the plurality of partial images are mapped, for each input image;
generating training data by acquiring a plurality of training partial images from the plurality of partial images based on the feature space;
training a prediction model for predicting a probability that a predetermined feature is included in the training partial image using the training data;
performing prediction for all or a part of the partial images included in the input image using a trained prediction model; and
acquiring a plurality of training partial images that are used as training data in a next training, based on predicted values for all or a part of the partial images in the feature space.

According to still another example aspect of the present invention, there is provided a recording medium recording a program, the program causing a computer to execute processing comprising:

generating a plurality of partial images smaller than an input image from the input image;
generating a feature space on which feature values of a plurality of partial images are mapped, for each input image;
generating training data by acquiring a plurality of training partial images from the plurality of partial images based on the feature space;
training a prediction model for predicting a probability that a predetermined feature is included in the training partial image using the training data;
performing prediction for all or a part of the partial images included in the input image using a trained prediction model; and

acquiring a plurality of training partial images that are used as training data in a next training, based on predicted values for all or a part of the partial images in the feature space.

EFFECT

According to the present disclosure, it is possible to perform training of a model with high accuracy while selecting effective areas as training data in a situation in which detailed labels are not given for the whole image.

BRIEF DESCRIPTION OF THE DRAWINGS

[FIG. 1] FIG. 1 shows a training device according to a first example embodiment.
[FIG. 2] FIG. 2 is a block diagram showing a hardware configuration of the training device according to the first example embodiment.
[FIG. 3] FIG. 3 is a block diagram showing a functional configuration of the training device.
[FIG. 4] FIG. 4 is an explanatory diagram of a process of an image dividing unit and a training data generation unit.
[FIG. 5] FIG. 5 shows examples of a partial image.
[FIG. 6] FIG. 6 shows an example of dividing a feature space into multiple spatial areas by grid division.
[FIG. 7] FIG. 7 is an explanatory diagram of a method of generating training data.
[FIG. 8] FIG. 8 is a flowchart of training processing by the training device.
[FIG. 9] FIG. 9 shows a display example of area selection probabilities when the training ends.
[FIG. 10] FIG. 10 is a block diagram showing a functional configuration of a training device according to a modification.
[FIG. 11] FIGS. 11A and 11B are diagrams for explaining processing of the training device according to the modification.
[FIG. 12] FIG. 12 is a block diagram showing a functional configuration of a prediction device.
[FIG. 13] FIG. 13 is a flowchart of prediction processing by the prediction device.
[FIG. 14] FIG. 14 is a block diagram showing a functional configuration of a training device of a second example embodiment.
[FIG. 15] FIG. 15 is a flowchart of processing by the training device of the second example embodiment.

EXAMPLE EMBODIMENTS

Preferred example embodiments of the present invention will be described with reference to the accompanying drawings.

<Basic principle>

In the present disclosure, in a device which predicts a predetermined image feature included in an input image, efficient training of a model is performed by selecting effective partial images based on a probability distribution on a feature space. In the following example embodiments, description will be given of an example in which a pathological tissue image of a patient on medication is used as an input and a medicinal effect is predicted based on the morphological features of the pathological tissue.

Specifically, at the time of training, the training device divides an input image into a plurality of partial images and trains a prediction model that performs prediction for each partial image. Here, the training device maps the plurality of partial images onto a feature space, and divides the feature space into a plurality of areas (hereinafter, referred to as "spatial areas") in which morphological features are mutually similar. Then, the training device generates training data by acquiring the partial images from the spatial areas according to the probability distribution assigned to the spatial areas, and trains the prediction model. Thus, it is possible to efficiently select the partial images effective for training from the input image to perform training of the prediction model. Further, when the training is performed and a trained model is obtained, the training device performs prediction for each partial image included in the input image using the trained prediction model, and updates the probability distribution of the plurality of spatial areas based on the prediction result. Then, the training device acquires the partial images from the spatial areas according to the updated probability distribution to generate training data, and further performs training of the prediction model. Thus, a highly accurate prediction model is generated by repeating the training of the prediction model while updating the probability distribution assigned to the plurality of spatial areas.

On the other hand, at the time of prediction using the trained prediction model (i.e., at the time of inference), the prediction device divides the input image into a plurality of partial images, performs prediction for the partial images using the trained prediction model, and integrates the prediction result for each partial image to obtain the prediction result for the input image. In addition, the prediction device can present a part of the input image, which was important for prediction, based on the prediction result for each partial image.

<First example embodiment>

[Training device]

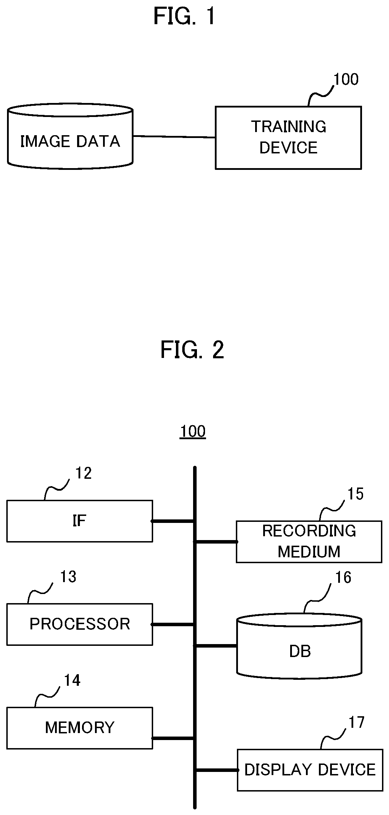

FIG. 1 shows a training device according to a first example embodiment. The training device 100 performs training of the prediction model based on the inputted image data (hereinafter, referred to as "input image").

(Hardware configuration)

FIG. 2 is a block diagram illustrating a hardware configuration of the training device 100 according to the first example embodiment. As shown, the training device 100 includes an interface (IF) 12, a processor 13, a memory 14, a recording medium 15, a database (DB) 16, and a display device 17.

The IF 12 inputs image data used to train a prediction model. The processor 13 is a computer such as a CPU (Central Processing Unit) and controls the entire training device 100 by executing a program prepared in advance. The processor 13 may be a GPU (Graphics Processing Unit) or a FPGA (Field-Programmable Gate Array). Specifically, the processor 13 executes training processing and prediction processing to be described later.

The memory 14 includes a ROM (Read Only Memory) and a RAM (Random Access Memory). The memory 14 stores various programs executed by the processor 13. The memory 14 is also used as a working memory during various processes performed by the processor 13.

The recording medium 15 is a non-volatile and non-transitory recording medium such as a disk-like recording medium, a semiconductor memory, or the like, and is configured to be detachable from the training device 100. The recording medium 15 records various programs executed by the processor 13. When the training device 100 executes various kinds of processes, the program recorded on the recording medium 15 is loaded into the memory 14 and executed by the processor 13.

The DB 16 stores the image data inputted through the IF 12. Specifically, the DB 16 stores the data of the input images used for the training by the training device 100. The display device 17 is, for example, a liquid crystal display device or a projector, and displays a prediction result by the training device 100. In addition to the above, the training device 100 may include an input device such as a keyboard and a mouse for the user to perform instructions and inputs.

(Functional configuration)

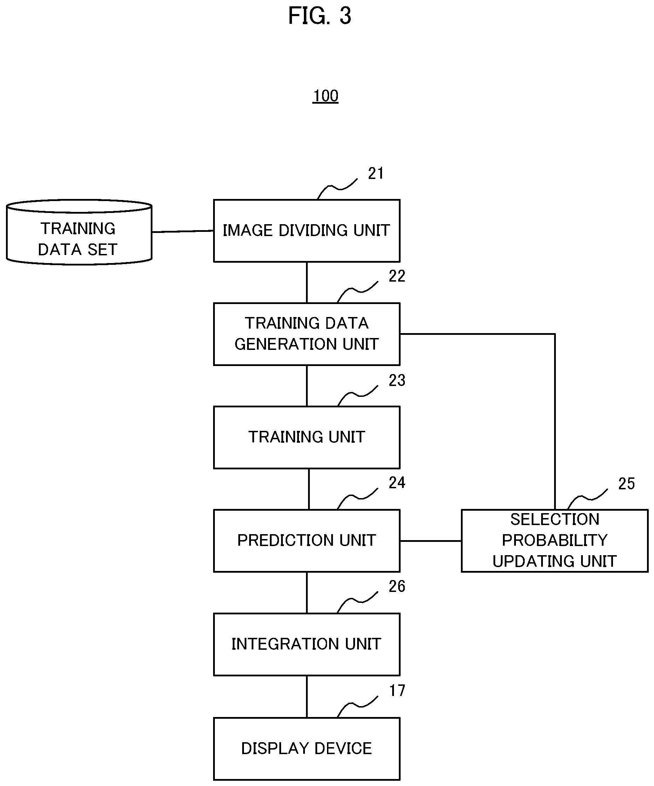

FIG. 3 is a block diagram showing a functional configuration of the training device. The training device 100 functionally includes an image dividing unit 21, a training data generation unit 22, a training unit 23, a prediction unit 24, a selection probability updating unit 25, and an integration unit 26. The output of the integration unit 26 is supplied to the display device 17.

At the time of training, a training data set is prepared. **In** the following description, a set of an input image and a teacher label (hereinafter, simply referred to as "label") for the input image is referred to as training data. The labels for input images are assigned in units of the input images. That is, one label is assigned to one whole input image, such that a positive label is given to an input image and a negative label is given to another input image. A set of training data for a plurality of input images is called "a training data set".

The training data generation unit 22, the training unit 23, the prediction unit 24, and the selection probability updating unit 25 are configured to repeat the loop processing (hereinafter, referred to as "training loop") for generating training data and training the prediction model for a predetermined number of times.

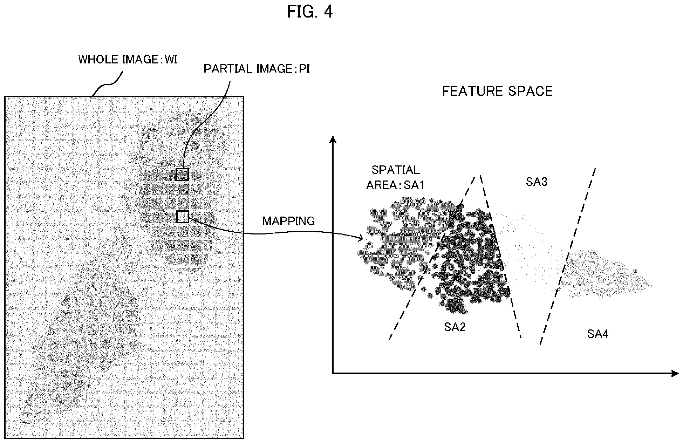

The image dividing unit 21 divides the input image included in the training data set into partial images smaller than the input image. The whole of one input image will be hereinafter referred to as the "whole image". FIG. 4 is an explanatory diagram of a process of the image dividing unit 21 and the training data generation unit 22. As shown in FIG. 4, the image dividing unit 21 divides the whole image WI into a plurality of partial images PI and outputs them to the training data generation unit 22. In the example of FIG. 4, the whole image WI is an image of the patient's pathological tissue. It is necessary for the image dividing unit 21 to generate the partial images for the pathological tissue region, but there is no need to generate the partial images for the background area. In the example of FIG. 4, the image dividing unit 21 divides the whole image WI into a plurality of partial images PI by grid division. Instead, the image dividing unit 21 may divide the whole image WI into a plurality of partial images PI centered on a certain characteristic point.

The training data generation unit 22 generates training data for training the prediction model using the plurality of partial images PI inputted. Specifically, the training data generation unit 22 first converts each of the plurality of partial images PI into a feature value to perform dimensional reduction and then maps them onto a two-dimensional feature space. FIG. 4 shows an example of mapping the partial images PI onto the feature space. One partial image PI is mapped as one point onto the two-dimensional feature space. As an algorithm for reducing dimensions, t-SNE(t-distributed Stochastic Neighbor Embedding), or UMAP (Uniform Manifold Approximation and Projection) can be used.

Next, the training data generation unit 22 divides the two-dimensional feature space into a plurality of areas (hereinafter also referred to as a "spatial area" in order to distinguish them from the regions within the image). There area two methods for dividing the two-dimensional feature space into multiple spatial areas. In the first method, the training data generation unit 22 divides the feature space into a plurality of spatial areas by clustering. In the example of FIG. 4, the training data generation unit 22 divides the feature space into four spatial areas SA1 to SA4 by clustering. Incidentally, for clustering, it is possible to use a known clustering technique such as kmeans, for example.

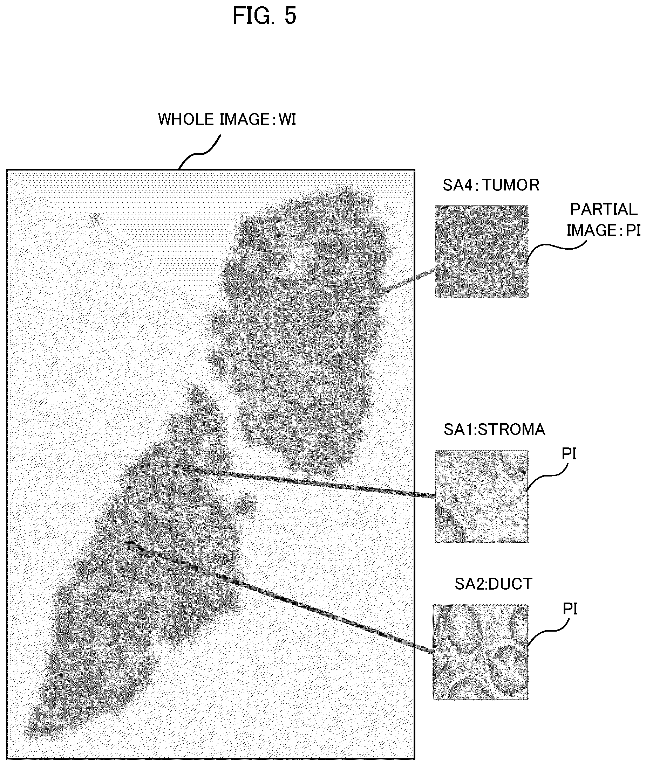

Since the points close to each other on the feature space shows that the image features of the partial images corresponding to those points are close to each other. Therefore, a plurality of partial images belonging to the same spatial area SA are the images of the tissues morphologically similar to each other. FIG. 5 shows examples of the partial image PI. If some partial images belonging to the spatial area SA1 are tissue images of stroma, other partial images belonging to the spatial area SA1 are also likely to be the tissue images of stroma. Similarly, if some partial images belonging to the spatial area SA2 are tissue images of a duct, other partial images belonging to the spatial area SA2 are also likely to be the tissue images of a duct. Further, if some partial images belonging to the spatial area SA4 are tissue images of tumor, other partial images belonging to the spatial area SA4 are also likely to be the tissue images of tumor.

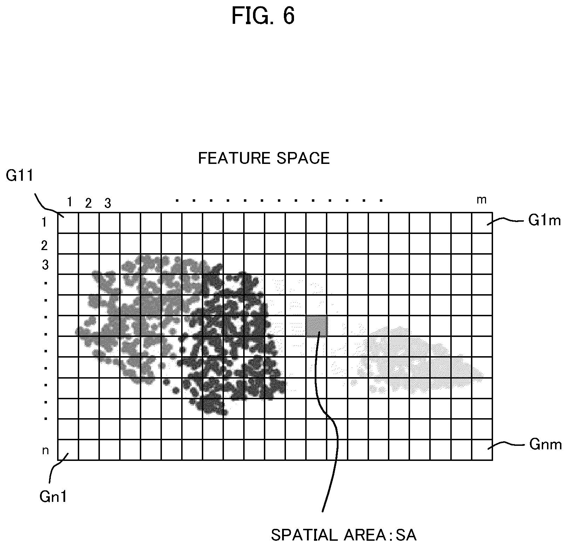

In the second method, instead of clustering, the training data generation unit 22 divides the feature space into a plurality of spatial areas by simple grid division. FIG. 6 shows an example in which the feature space is divided into a plurality of spatial areas by grid division. In this example, the whole image WI is divided into n×m grids. In this case, each grid corresponds to one spatial area.

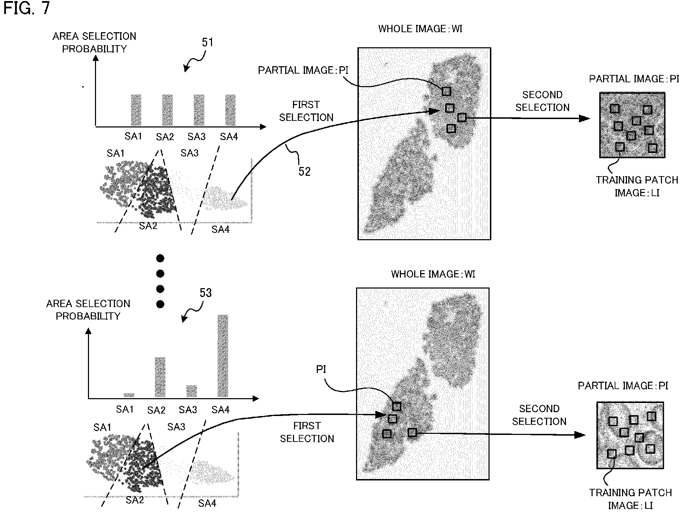

Next, the training data generation unit 22 acquires the partial images from the plurality of spatial areas obtained by the above-described first or second method and generates the training data. FIG. 7 is an explanatory diagram of a method of generating training data. In the following description, it is assumed that the first method described above is used. First, the training data generation unit 22 sets a probability (hereinafter, referred to as "area selection probability") of selecting one spatial area used for generating the training data from the plurality of spatial areas. For example, the area selection probability of the spatial area SA1 indicates the probability that the spatial area SA1 is selected from the four spatial areas SA1 to SA4.

As shown in FIG. 7, in the initial state, the training data generation unit 22 sets the area selection probabilities of the spatial areas to the same value. For example, the training data generation unit 22 sets the area selection probabilities of the spatial areas SA1 to SA4 to the same value, as shown in a graph 51 of the probability distribution. Then, the training data generation unit 22 selects one spatial area from the spatial areas SA1 to SA4 based on the set area selection probabilities (first selection). In the example of FIG. 7, as shown by an arrow 52, the training data generation unit 22 selects the spatial area SA4.

Next, the training data generation unit 22 selects a predetermined number of partial images PI from the plurality of partial images PI belonging to the selected spatial area (second selection). Then, the training data generation unit 22 generates the partial images (hereinafter, also referred to as "patch images") by further subdividing each of the selected partial images PI by the grid division or the like. Then, the training data generation unit 22 acquires the patch images used for training (hereinafter, referred to as "training patch images") from each of the partial images PI. Specifically, the training data generation unit 22 may acquire all the patch images obtained by subdividing the partial image PI as the training patch images LI, or may acquire a predetermined number of patch images from all the patch images as the training patch images LI by selecting the predetermined number of patch images by random sampling. The training patch image is an example of a training partial image. Thus, the training patch images LI are generated based on the plurality of partial images corresponding to the selected spatial area (the spatial area SA4 in FIG. 7).

Next, the training data generation unit 22 assigns labels to the obtained training patch images LI. As described above, each training data included in the training data set is labeled for each input image, i.e., for each whole image WI. Therefore, the training data generation unit 22 uses the label given to the whole image WI to which each partial image belongs as the label of each training patch image LI generated from the partial image. In this way, the training data generation unit 22 assigns labels to all the training patch images LI and ends the generation of the training data. The training data generation unit 22 generates training data for the plurality of input images.

The training unit 23 performs training of the prediction model using the training data inputted from the training data generation unit 22. The prediction model predicts the probability that a predetermined image feature is included in the input image (the whole image WI). In the present example embodiment, the prediction model predicts the probability that a predetermined feature such as the aforementioned tumor, stroma, or duct is included in the patient's pathological tissue image, and outputs the confidence level (score) for each feature. As the prediction model, a deep learning model such as CNN (Convolutional Neural Network) can be used. The training unit 23 performs the first training using the inputted training data and outputs the trained prediction model to the prediction unit 24.

The prediction unit 24 performs prediction for all the partial images constituting the input image using the trained prediction model, calculates the prediction values and outputs them to the selection probability updating unit 25 and the integration unit 26. The prediction unit 24 performs this process for all the input images included in the training data set.

The selection probability updating unit 25 calculates a prediction result corresponding to each spatial area on the basis of the prediction values outputted by the prediction unit 24 using the trained prediction model. Specifically, the selection probability updating unit 25 calculates the average value of the predicted values for the partial images included in the spatial area SA1 as the prediction result for the spatial area SA1. For example, the selection probability updating unit 25 sets the prediction result to "+1" if the average value is equal to or larger than a predetermined threshold value and sets the prediction result to "-1" if the average value is smaller than the threshold value. The selection probability updating unit 25 performs this process in the same manner for other spatial areas SA2 to SA4, and generates the prediction result for each spatial area.

Next, the selection probability updating unit 25 updates the area selection probability of each spatial area using the prediction result for each spatial area. Specifically, the selection probability updating unit 25 updates the area selection probability such that the spatial area for which the reliability of the prediction result by the prediction model is high is more likely to be selected in the next training data.

In a preferred example, the selection probability updating unit 25 calculates the selection probability $D_{t+1}$ (i) of the i-th spatial area SAi by using the following equation (1).

[Equation 1]

$$D_{t+1}(i) = \frac{D_t(i)e^{\alpha_t y_i h_t(x_i)}}{\sum_j e^{\alpha_t y_j h_t(x_j)}} \qquad (1)$$

Here, "t" is the number of iterations of the training loop. "$\alpha_t$" indicates the weight of the model after the t-th training, and is expressed by the following equation (2):

[Equation 2]

$$\alpha_t = \log\left(\frac{1 - \varepsilon_t}{\varepsilon_t}\right) \qquad (2)$$

Also, "$\varepsilon_t$" is the error probability of the model after the t-th training, and is expressed by the following equation (3).

[Equation 3]

$$\varepsilon_t = \sum_{y_i \neq h_t(x_i)} D_t(i) \qquad (3)$$

Also, "$h_t(x_i)$" is the prediction result of the model for the i-th spatial area SAi ($\pm 1$), and "$y_i$" is the label of the i-th spatial area SAi ($\pm 1$). By repeating the training loop while updating the area selection probabilities using the above equation (1), the error probability of the model decreases.

The selection probability updating unit 25 outputs the area selection probabilities of the spatial areas after the updating to the training data generation unit 22. The training data generation unit 22 generates the training data to be used for the next training by using the inputted area selection probabilities after the updating.

Thus, the training loop is repeated a predetermined number of times while updating the area selection probability of each spatial area. Then, when the training loop is repeated the predetermined number of times, the prediction unit 24 performs prediction for all the partial images included in the input image using the trained model at the time of ending the training (also referred to as "final model"), and outputs the prediction values to the integration unit 26.

The integration unit 26 integrates the predicted values for each partial image outputted by the prediction unit 24 and generates the prediction result for the whole input image. For example, for each input image, the integration unit 26 sets the average value of the predicted values of all the partial images constituting the input image as the predicted value of the whole input image. Then, the integration unit 26 displays the predicted value of the whole input image on the display device 17 together with the input image.

In the above-described configuration, the image dividing unit 21 is an example of the partial image generation means, the training data generation unit 22 is an example of the training data generation means, the training unit 23 is an example of the training means, and the prediction unit 24 is an example of the prediction means.

(Training processing)

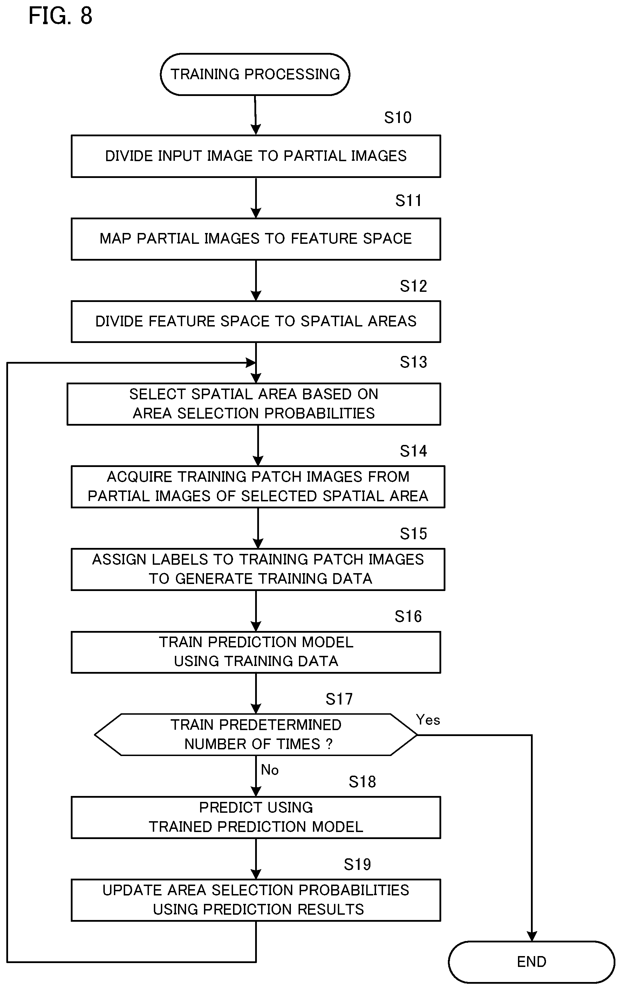

FIG. 8 is a flowchart of training processing performed by the training device 100. This processing is realized by the processor 13 shown in FIG. 2, which executes a pre-prepared program and operates as the elements shown in FIG. 3.

First, the image dividing unit 21 receives the training data set, and divides the respective input images included in the training data set into the partial images (step S10). Next, the training data generation unit 22 maps the partial images onto the feature space for each input image (step S11). Next, the training data generation unit 22 divides the feature space into a plurality of spatial areas (step S12). At this time, the training data generation unit 22 may divide the feature space by clustering as described above or may divide the feature space by grid division.

Next, the training data generation unit 22 selects one spatial area based on the set area selection probabilities for the respective spatial areas (step S13). Incidentally, in the initial state, the area selection probabilities of the spatial areas are set to the same value. Next, the training data generation unit 22 generates the training patch images from a predetermined number of partial images belonging to the selected spatial area (step S14). Next, the training data generation unit 22 assigns labels to the respective training patch images and generates the training data (step S15).

Next, the training unit 23 trains the prediction model using the training data generated in step S15 and generates a trained prediction model (step S16). Next, the prediction unit 24 determines whether or not the training unit 23 has trained the prediction model the predetermined number of times (step S14). That is, the prediction unit 24 determines whether or not the above-described training loop has been repeated the predetermined number of times. When the training unit 23 has not trained the prediction model the predetermined number of times (step S17: No), the prediction unit 24 performs prediction for the partial images included in all the input images in the training data set using the trained prediction model generated in step S16 (step S18).

Next, the selection probability updating unit 25 updates the area selection probability of each spatial area based on the prediction result of each spatial area by the prediction unit 24 (step S19). Thus, the higher the reliability of the prediction result by the trained prediction model for the spatial area is, the higher the area selection probability for the spatial area becomes, and it becomes more likely that the spatial area is selected in the generation of the training data in the next time. Then, the process returns to step S13.

Thus, the training loop of steps S13 to S19 is repeated until the training unit 23 performs training the predetermined number of times. Then, when the training unit 23 completes the training the predetermined number of times (step S14: Yes), the training processing ends.

(Presentation of basis for prediction by the model)

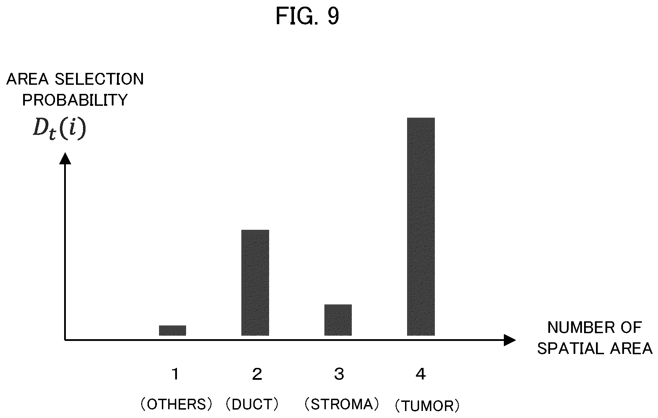

By displaying the area selection probabilities at the end of the above-mentioned training on the display device 17, it becomes possible to present the basis of the prediction by the prediction model. FIG. 9 shows a display example of the area selection probabilities at the end of the training. **In** this example, for each spatial area, the tissue corresponding to that spatial area and the area selection probability of that spatial area are displayed. By this display, it is possible to show at what frequency the tissue corresponding to each spatial area was selected in the training of the prediction model. For example, if the prediction model is a model that predicts the medicinal effect for each tissue, it is possible to know which tissue the medicinal effect is large.

(Modification)

Next, a modification of the training device according to the first example embodiment will be described. As shown in FIG. 4, the above-described training device 100 divides the whole of the pathological tissue region included in the whole image WI into a plurality of partial images PI and maps them onto the feature space. This is effective in ensuring the diversity of the pathological tissues indicated by the partial images PI. However, since a normal region or a region apparently unrelated to cancer is mapped, wasteful process may be increased.

Therefore, in this modification, the training device detects tumor cells, lymphocytes, or the like from the pathological tissue region included in the whole image WI using the tumor cell detection method, and selects and maps the partial images PI including the tumor cells with higher possibility onto the feature space. This enables to reduce the calculation amount by narrowing down the objects of mapping onto the feature space to the partial images of the cancers and their surroundings (regions that are likely to be highly related to medicinal effect).

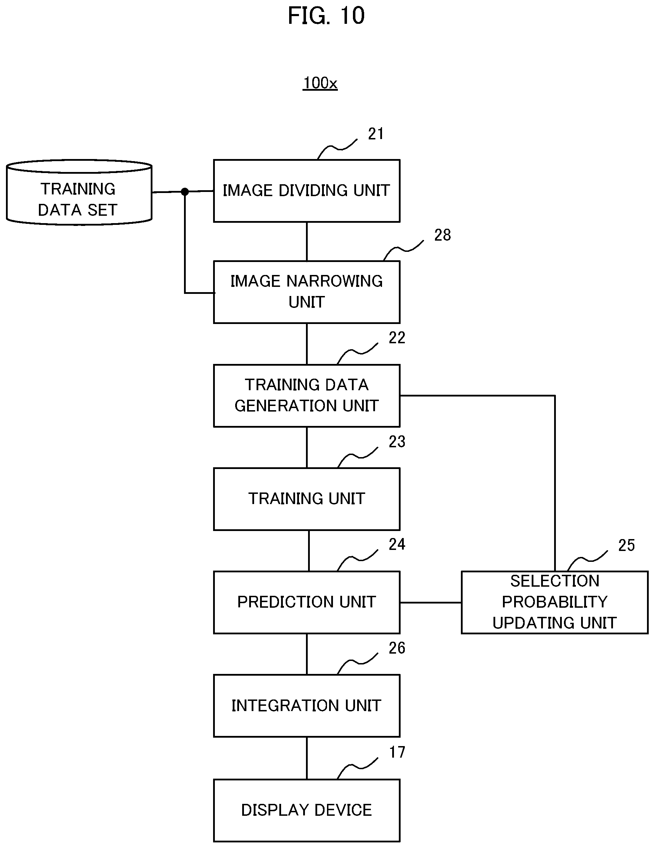

FIG. 10 shows a functional configuration of a training device 100x according to the modification. As understood by the comparison with FIG. 3, in the training device 100x, an image narrowing unit 28 is provided between the image dividing unit 21 and the training data generation unit 22. The image narrowing unit 28 performs a tumor cell detecting process on the whole image WI and detects tumor cells or the like. Existing tumor cell detection techniques can be used for the detection of tumor cells. An example of a tumor cell detection technique is described in the following document

Cosatto, E., Gerard, K., Graf, H. P., Ogura, M., Kiyuna, T., Hatanaka, K. C., ... & Hatanaka, Y. (2021, February). A multi-scale conditional deep model for tumor cell ratio counting. In Medical Imaging 2021: Digital Pathology (Vol. 11603, pp. 31-42). SPIE.

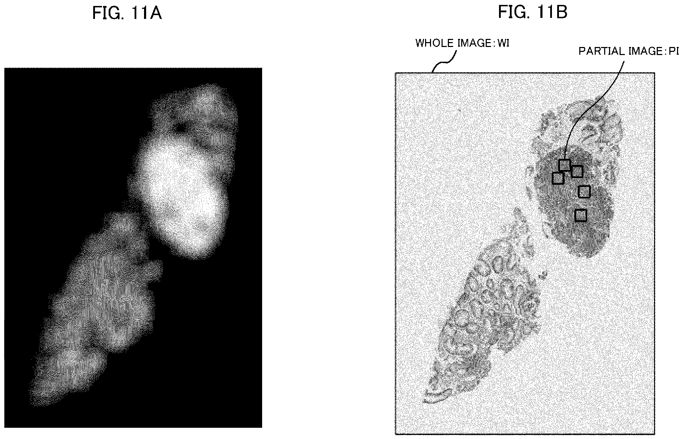

FIG. 11A shows an example of detecting tumor cells using an existing tumor cell detection technique. FIG. 11A shows the density of the tumor cells in grayscale. The higher the brightness is, the higher the proportion of the tumor cells is. The image narrowing unit 28 selects the partial images PI from the regions having the higher local peaks in the density distribution of the tumor cells on the basis of the detection result of the tumor cells as exemplified in FIG. 11A and outputs the selected partial images PI to the training data generation unit 22. Specifically, as shown in FIG. 11B, the image narrowing unit 28 selects the partial images PI from the region including the tumor cells with a high proportion, i.e., the region having a high brightness in FIG. 11A, and outputs them to the training data generation unit 22 as the objects of the mapping onto the feature space. This makes it possible to improve the accuracy of the model while reducing the calculation load.

[Prediction device]

Next, a prediction device that performs prediction using the trained model generated by the training device will be described.

(Hardware configuration)

The hardware configuration of the prediction device is basically the same as the hardware configuration of the training device 100 shown in FIG. 2. However, unlike the training device 100, the IF 12 acquires an input image subjected to the prediction from a data base or the like, and the display device 17 displays a prediction result for the input image subjected to the prediction.

(Functional configuration)

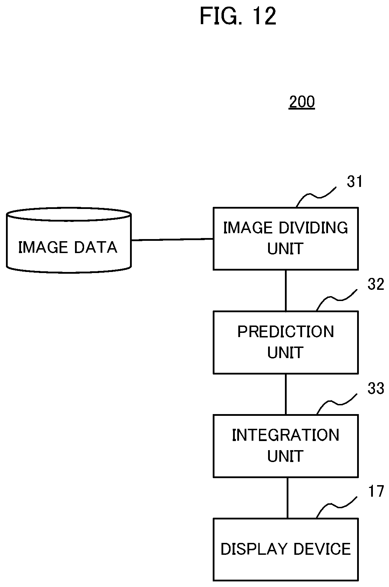

FIG. 12 is a block diagram illustrating a functional configuration of the prediction device. The prediction device 200 functionally includes an image dividing unit 31, a prediction unit 32, and an integration unit 33. The output of the integration unit 33 is supplied to the display device 17.

At the time of actual prediction, the input image subjected to the prediction is prepared. In the present example embodiment, as the input image subjected to the prediction, the pathological tissue image of the patient on medication is inputted to the image dividing unit 31. The image dividing unit 31 divides the input image, i.e., the whole image WI, into a plurality of partial images PI in the same manner as in the training, and further divides each partial image PI into patch images of the same size as the training patch images LI (hereinafter referred to as "prediction patch images"). That is, the image dividing unit 31 divides the input image subjected to the prediction into the size suitable for the input to the trained prediction model. Then, the image dividing unit 31 outputs the prediction patch images obtained by the division to the prediction unit 32.

The prediction unit 32 performs prediction for the input image using the trained prediction model obtained by the above-described training processing. Specifically, the prediction unit 32 performs prediction, using the trained prediction model, for each prediction patch image obtained by the image dividing unit 31 and outputs the prediction values to the integration unit 33.

The integration unit 33 calculates the predicted value of the whole input image by integrating the predicted values calculated by the prediction unit 32 for each prediction patch image, and outputs the predicted value of the whole input image to the display device 17. The integration unit 33 may output the calculated prediction value to the external device. This yields the probability that a tissue with a predetermined feature (in the previous example, a tumor, a stroma, a duct, etc.) is included in the inputted pathological tissue image. Then, the display device 17 displays the predicted value and the important region on the input image. Also, in the prediction for the input image, the integration unit 33 may extract the region of the partial image in which the predicted value is equal to or larger than a predetermined reference value as an important region for the prediction, and may display it on the display device 17. In this way, the prediction result for the input image is outputted.

The prediction model used by the prediction unit 32 is basically the prediction model at the time when the training loop is repeated a predetermined number of times, i.e., the final model. However, since the accuracy of the final model is not always the highest, one of the prediction models having the smallest prediction error among the prediction models obtained in each training loop may be used in the prediction unit 32. Specifically, the prediction error may be calculated by comparing the prediction result by the prediction model obtained at the end of each training loop with the training data set, and the prediction model with the smallest prediction error may be adopted.

In addition, in the above example, one of the plurality of prediction models obtained by the repetition of the training loop is used for prediction in the prediction unit 32. Instead, some of the prediction models obtained may be used in combination. Specifically, the prediction unit 32 may perform prediction for the input image using the prediction models obtained by the repetition of the training loop, and may output a final prediction result by weighting and adding the obtained prediction results. In this case, it is preferable to give a greater weight to the output of the predictive model with higher accuracy.

(Inference processing)

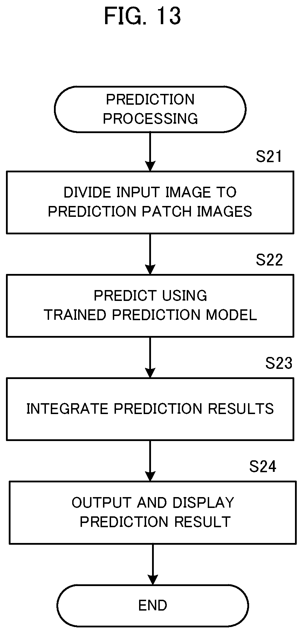

FIG. 13 is a flowchart of prediction processing performed by the prediction device 200. This processing is realized by the processor 13 shown in FIG. 2, which executes a pre-prepared program and operates as the elements shown in FIG. 12.

First, the image dividing unit 31 divides the input image into partial images, and further divides the partial images into predictive patch images (step S21). Next, the prediction unit 32 performs prediction for the respective prediction patch images using the trained prediction model obtained by the training processing and outputs the prediction values (step S22). Next, the integration unit 33 integrates the prediction values for the respective prediction patch images and calculates the prediction result for the whole input image (step S23). Then, the display device 17 displays the prediction result (step S24). Then, the prediction processing ends.

[Application example]

Next, description will be given of details of an example in which the prediction device of the present example embodiment is applied to the prediction of the effect of medication (medicinal effect or success, hereinafter referred to as "medicinal effect"). In the field of the medical treatment, the medicinal effect is predicted based on the image using the pathological tissue image as an input. For example, there is a method to predict the medicinal effect based on the staining rate of immune images using the image in which cells containing the pathological tissue are stained. In this case, there are problems that the determination of the staining rate varies depending on the inspector and that it is not clearly known which part in the image is a characteristic part reflecting the medicinal effect.

Therefore, by using the pathological tissue image as an input, the prediction device of the present example embodiment predicts the medicinal effect using the prediction model obtained by the training, thereby to output the score of the medicinal effect and display an area important for the determination of the medicinal effect.

Specifically, at the time of training, the training data in which the label indicating the presence or absence of the medicinal effect is given to the pathological tissue image is prepared, and the above-described training processing is executed to train the prediction model of the medicinal effect. At this time, since it is sufficient to perform labeling to the whole of the pathological tissue image in the method of the present example embodiment, the labeling can be performed even if it is not clear which part in the pathological tissue image is reflecting the medicinal effect. Then, at the time of prediction, by inputting a pathological tissue image subjected to the prediction, the score of the medicinal effect can be predicted using the trained prediction model. In addition, the region of the partial image which showed the high score in the prediction processing can be extracted as an important region in which the influence on the medicinal effect is large, and the region can be displayed on the display device.

When this example embodiment is applied to the prediction of the medicinal effect, it is preferable that the image dividing unit 21 uses background knowledge at the time of training and prediction such that information around the cell nucleus is particularly important for diagnosis, and generates the partial images around the position of the cell nucleus in the input image.

<Second example embodiment>

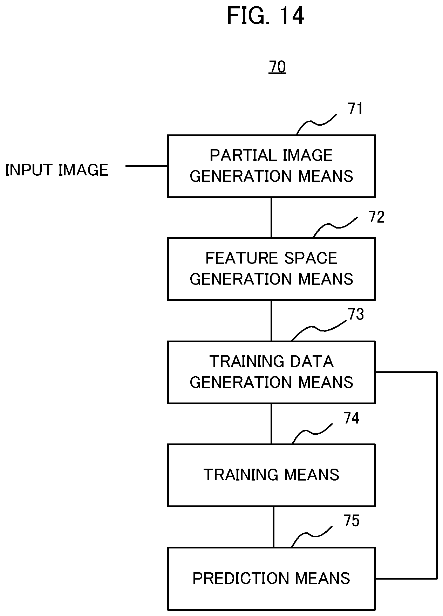

FIG. 14 is a block diagram illustrating a functional configuration of a training device according to a second example embodiment. The training device 70 includes a partial image generation means 71, a feature space generation means 72, a training data generation means 73, a training means 74, and a prediction means 75.

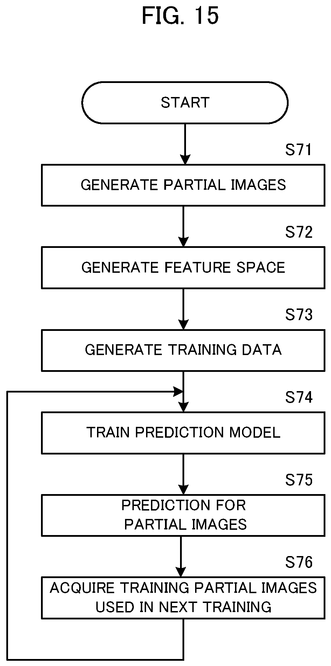

FIG. 15 is a flowchart of processing performed by the training device 70 according to the second example embodiment. The partial image generation means 71 generates a plurality of partial images smaller than an input image from the input image (step S71). The feature space generation means 72 generates a feature space to which feature values of the plurality of partial images are mapped, for each input image (step S72). The training data generation means 73 generates training data by acquiring a plurality of training partial images from the plurality of partial images based on the feature space (step S73). The training means 74 trains a prediction model for predicting a probability that a predetermined feature is included in the training partial image using the training data (step S74). The prediction means 75 performs prediction for all or a part of the partial images included in the input image using a trained prediction model (step S75). Further, the training data generation means 73 acquires a plurality of training partial images that are used as training data in a next training, based on predicted values for all or a part of the partial images in the feature space (step S76). Thus, the training of the prediction model by the training means 74 is repeated while the training partial images are updated.

According to the training device 70 of the second example embodiment, it becomes possible to perform training of a model with high accuracy while selecting an effective area as training data, under the situation in which a detailed label is not given to the whole image.

A part or all of the example embodiments described above may also be described as the following supplementary notes, but not limited thereto.

(Supplementary note 1)

A training device comprising:

a partial image generation means configured to generate a plurality of partial images smaller than an input image from the input image;
a feature space generation means configured to generate a feature space to which feature values of the plurality of partial images are mapped, for each input image;
a training data generation means configured to generate training data by acquiring a plurality of training partial images from the plurality of partial images based on the feature space;
a training means configured to train a prediction model for predicting a probability that a predetermined feature is included in the training partial image using the training data; and
a prediction means configured to perform prediction for all or a part of the partial images included in the input image using a trained prediction model,
wherein the training data generation means acquires a plurality of training partial images that are used as training data in a next training, based on predicted values for all or a part of the partial images in the feature space.

(Supplementary note 2)

The training device according to Supplementary note 1, wherein the training data generation means comprises:

a spatial area dividing means configured to divide the feature space into a plurality of spatial areas; and
an acquisition means configured to determine selection probabilities of the plurality of spatial areas, and acquires the plurality of training partial images from the plurality of partial images corresponding to the spatial area selected according to the selection probabilities.

(Supplementary note 3)

The training device according to Supplementary note 2, wherein the training data generation means updates the selection probabilities of the plurality of spatial areas based on predicted values for all or a part of the partial images included in the input image.

(Supplementary note 4)

The training device according to Supplementary note 3, wherein the training data generation means updates the selection probabilities of the plurality of spatial areas such that, as reliability of the predicted value for the partial image is high, the selection probability of the spatial area corresponding to the partial image becomes high.

(Supplementary note 5)

The training device according to Supplementary note 2, wherein the spatial area dividing means divides the feature space into the plurality of spatial areas by mapping the feature values of the plurality of partial images onto the feature space and clustering distribution of the feature values.

(Supplementary note 6)

The training device according to Supplementary note 1, wherein the training data generation means uses a label given to the input image in advance, as the label for each training partial image acquired from the input image.

(Supplementary note 7)

The training device according to Supplementary note 1, further comprising an output means configured to output the selection probabilities of the plurality of spatial areas at a time of ending the training of the trained prediction model.

(Supplementary note 8)

The training device according to Supplementary note 1, wherein the feature space generation means selects the partial images in which a proportion of tumor cells is high, from among the plurality of partial images generated from the input image, and maps the selected partial images onto the feature space.

(Supplementary note 9)

A prediction device comprising:

a partial image generation means configured to generate partial images smaller than an input image from the input image;

a prediction means configured to predict a probability that a predetermined feature is included in the partial image, for all the generated partial images, using a prediction model trained by the training device according to any one of Supplementary notes 1 to 7; and

an output means configured to integrate prediction results for all the partial images and output a prediction score indicating a probability that the predetermined feature is included in the input image.

(Supplementary note 10)

A training method executed by a computer, comprising:

generating a plurality of partial images smaller than an input image from the input image;

generating a feature space on which feature values of the plurality of partial images are mapped, for each input image;

generating training data by acquiring a plurality of training partial images from the plurality of partial images based on the feature space;

training a prediction model for predicting a probability that a predetermined feature is included in the training partial image using the training data;

performing prediction for all or a part of the partial images included in the input image using a trained prediction model; and

acquiring a plurality of training partial images that are used as training data in a next training, based on predicted values for all or a part of the partial images in the feature space.

(Supplementary note 11)

A recording medium recording a program, the program causing a computer to execute processing comprising:

generating a plurality of partial images smaller than an input image from the input image;

generating a feature space on which feature values of a plurality of partial images are mapped, for each input image;

generating training data by acquiring a plurality of training partial images from the plurality of partial images based on the feature space;

training a prediction model for predicting a probability that a predetermined feature is included in the training partial image using the training data;

performing prediction for all or a part of the partial images included in the input image using a trained prediction model; and

acquiring a plurality of training partial images that are used as training data in a next training, based on predicted values for all or a part of the partial images in the feature space.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

DESCRIPTION OF SYMBOLS

13 Processor
17 Display device
21, 31 Image dividing unit
22 Training data generation unit
23 Training unit
24, 32 Prediction unit
25 Selection probability updating unit
26,33 Integration unit
100 Training device
200 Prediction device

**Claims**

1. A training device comprising:

   a partial image generation means configured to generate a plurality of partial images smaller than an input image from the input image;
   a feature space generation means configured to generate a feature space to which feature values of the plurality of partial images are mapped, for each input image;
   a training data generation means configured to generate training data by acquiring a plurality of training partial images from the plurality of partial images based on the feature space;
   a training means configured to train a prediction model for predicting a probability that a predetermined feature is included in the training partial image using the training data; and
   a prediction means configured to perform prediction for all or a part of the partial images included in the input image using a trained prediction model,
   wherein the training data generation means acquires a plurality of training partial images that are used as training data in a next training, based on predicted values for all or a part of the partial images in the feature space.

2. The training device according to claim 1, wherein the training data generation means comprises:

   a spatial area dividing means configured to divide the feature space into a plurality of spatial areas; and
   an acquisition means configured to determine selection probabilities of the plurality of spatial areas, and acquires the plurality of training partial images from the plurality of partial images corresponding to the spatial area selected according to the selection probabilities.

3. The training device according to claim 2, wherein the training data generation means updates the selection probabilities of the plurality of spatial areas based on predicted values for all or a part of the partial images included in the input image.

4. The training device according to claim 3, wherein the training data generation means updates the selection probabilities of the plurality of spatial areas such that, as reliability of the predicted value for the partial image is high, the selection probability of the spatial area corresponding to the partial image becomes high.

5. The training device according to claim 2, wherein the spatial area dividing means divides the feature space into the plurality of spatial areas by mapping the feature values of the plurality of partial images onto the feature space and clustering distribution of the feature values.

6. The training device according to claim 1, further comprising an output means configured to output the selection probabilities of the plurality of spatial areas at a time of ending the training of the trained prediction model.

7. The training device according to claim 1, wherein the feature space generation means selects the partial images in which a proportion of tumor cells is high, from among the plurality of partial images generated from the input image, and maps the selected partial images on the feature space.

8. A prediction device comprising:

   a partial image generation means configured to generate partial images smaller than an input image from the input image;
   a prediction means configured to predict a probability that a predetermined feature is included in the partial image, for all the generated partial images, using a prediction model trained by the training device according to any one of claims 1 to 7; and
   an output means configured to integrate prediction results for all the partial images and output a prediction score indicating a probability that the predetermined feature is included in the input image.

9. A training method executed by a computer, comprising:

generating a plurality of partial images smaller than an input image from the input image;
generating a feature space on which feature values of the plurality of partial images are mapped, for each input image;
generating training data by acquiring a plurality of training partial images from the plurality of partial images based on the feature space;
training a prediction model for predicting a probability that a predetermined feature is included in the training partial image using the training data;
performing prediction for all or a part of the partial images included in the input image using a trained prediction model; and
acquiring a plurality of training partial images that are used as training data in a next training, based on predicted values for all or a part of the partial images in the feature space.

10. A recording medium recording a program, the program causing a computer to execute processing comprising:

generating a plurality of partial images smaller than an input image from the input image;
generating a feature space on which feature values of a plurality of partial images are mapped, for each input image;
generating training data by acquiring a plurality of training partial images from the plurality of partial images based on the feature space;
training a prediction model for predicting a probability that a predetermined feature is included in the training partial image using the training data;
performing prediction for all or a part of the partial images included in the input image using a trained prediction model; and
acquiring a plurality of training partial images that are used as training data in a next training, based on predicted values for all or a part of the partial images in the feature space.

## FIG. 1

IMAGE DATA

100

TRAINING
DEVICE

## FIG. 2

100

12

IF

13

PROCESSOR

14

MEMORY

15

RECORDING
MEDIUM

16

DB

17

DISPLAY DEVICE

# FIG. 3

100

FIG. 4

WHOLE IMAGE : WI    PARTIAL IMAGE : PI

FEATURE SPACE

SPATIAL
AREA : SA1

SA3

MAPPING

SA2

SA4

# FIG. 5

WHOLE IMAGE:WI

SA4:TUMOR

PARTIAL
IMAGE:PI

SA1:STROMA

PI

SA2:DUCT

PI

# FIG. 6

FEATURE SPACE

SPATIAL AREA : SA

FIG. 7

## FIG. 8

```
        ┌─────────────────────────┐
        │   TRAINING PROCESSING   │
        └─────────────────────────┘
                     │
                     ▼                    S10
        ┌─────────────────────────────────┐
        │ DIVIDE INPUT IMAGE TO PARTIAL   │
        │             IMAGES              │
        └─────────────────────────────────┘
                     │
                     ▼                    S11
        ┌─────────────────────────────────┐
        │ MAP PARTIAL IMAGES TO FEATURE   │
        │             SPACE               │
        └─────────────────────────────────┘
                     │
                     ▼                    S12
        ┌─────────────────────────────────┐
        │ DIVIDE FEATURE SPACE TO SPATIAL │
        │             AREAS               │
        └─────────────────────────────────┘
                     │
                     ▼                    S13
        ┌─────────────────────────────────┐
        │     SELECT SPATIAL AREA BASED ON │
        │     AREA SELECTION PROBABILITIES │
        └─────────────────────────────────┘
                     │
                     ▼                    S14
        ┌─────────────────────────────────┐
        │ ACQUIRE TRAINING PATCH IMAGES   │
        │ FROM PARTIAL IMAGES OF SELECTED │
        │         SPATIAL AREA            │
        └─────────────────────────────────┘
                     │
                     ▼                    S15
        ┌─────────────────────────────────┐
        │ ASSIGN LABELS TO TRAINING PATCH │
        │ IMAGES TO GENERATE TRAINING DATA │
        └─────────────────────────────────┘
                     │
                     ▼                    S16
        ┌─────────────────────────────────┐
        │     TRAIN PREDICTION MODEL      │
        │       USING TRAINING DATA       │
        └─────────────────────────────────┘
                     │
                     ▼            S17
              ╱─────────────────╲      Yes
             ╱ TRAIN PREDETERMINED ╲──────────┐
             ╲ NUMBER OF TIMES ?   ╱          │
              ╲─────────────────╱            │
                     │ No                     │
                     ▼        S18             │
        ┌─────────────────────────────┐       │
        │       PREDICT USING         │       │
        │  TRAINED PREDICTION MODEL   │       │
        └─────────────────────────────┘       │
                     │                        │
                     ▼            S19          │
        ┌─────────────────────────────────┐   │
        │ UPDATE AREA SELECTION           │   │
        │ PROBABILITIES USING PREDICTION  │   │
        │             RESULTS             │   │
        └─────────────────────────────────┘   │
                     │                        ▼
                     (loop back to S13)  ┌─────────┐
                                         │   END   │
                                         └─────────┘
```

# FIG. 9

AREA SELECTION
PROBABILITY
$D_t(i)$

NUMBER OF
SPATIAL AREA

1      2      3      4

(OTHERS) (DUCT) (STROMA) (TUMOR)

# FIG. 10

<u>100x</u>

FIG. 11A

FIG. 11B

WHOLE IMAGE：WI

PARTIAL IMAGE：PI

# FIG. 12

<u>200</u>

IMAGE DATA

IMAGE DIVIDING UNIT — 31

PREDICTION UNIT — 32

INTEGRATION UNIT — 33

DISPLAY DEVICE — 17

# FIG. 13

```
        ╭─────────────────╮
        │   PREDICTION    │
        │   PROCESSING    │
        ╰────────┬────────╯
                 │              S21
        ┌────────▼────────┐
        │ DIVIDE INPUT IMAGE TO   │
        │ PREDICTION PATCH IMAGES │
        └────────┬────────┘
                 │              S22
        ┌────────▼────────┐
        │   PREDICT USING         │
        │ TRAINED PREDICTION MODEL│
        └────────┬────────┘
                 │              S23
        ┌────────▼────────┐
        │ INTEGRATE PREDICTION RESULTS │
        └────────┬────────┘
                 │              S24
        ┌────────▼────────┐
        │  OUTPUT AND DISPLAY     │
        │  PREDICTION RESULT      │
        └────────┬────────┘
                 │
        ╭────────▼────────╮
        │      END        │
        ╰─────────────────╯
```

# FIG. 14

<u>70</u>

INPUT IMAGE ——— PARTIAL IMAGE GENERATION MEANS　71

FEATURE SPACE GENERATION MEANS　72

TRAINING DATA GENERATION MEANS　73

TRAINING MEANS　74

PREDICTION MEANS　75

# FIG. 15

```
        ┌─────────────────┐
        │      START      │
        └─────────────────┘
                 │
                 ▼              S71
        ┌─────────────────────┐
        │ GENERATE PARTIAL IMAGES │
        └─────────────────────┘
                 │
                 ▼              S72
        ┌─────────────────────┐
        │ GENERATE FEATURE SPACE │
        └─────────────────────┘
                 │
                 ▼              S73
        ┌─────────────────────┐
        │ GENERATE TRAINING DATA │
        └─────────────────────┘
                 │
                 ▼              S74
        ┌─────────────────────┐
   ┌───▶│ TRAIN PREDICTION MODEL │
   │    └─────────────────────┘
   │             │
   │             ▼              S75
   │    ┌─────────────────────┐
   │    │   PREDICTION FOR     │
   │    │   PARTIAL IMAGES     │
   │    └─────────────────────┘
   │             │
   │             ▼              S76
   │    ┌───────────────────────────┐
   │    │ ACQUIRE TRAINING PARTIAL IMAGES │
   │    │   USED IN NEXT TRAINING    │
   │    └───────────────────────────┘
   │             │
   └─────────────┘
```

## INTERNATIONAL SEARCH REPORT

| International application No. |
|---|
| **PCT/JP2022/043350** |

| A. | CLASSIFICATION OF SUBJECT MATTER |
|---|---|

*G06T 7/00*(2017.01)i
FI: G06T7/00 350B

According to International Patent Classification (IPC) or to both national classification and IPC

| B. | FIELDS SEARCHED |
|---|---|

Minimum documentation searched (classification system followed by classification symbols)

G06T7/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2018/116921 A1 (NEC CORPORATION) 28 June 2018 (2018-06-28)<br>entire text, all drawings | 1-10 |
| A | JP 2022-539791 A (STRADVISION INC) 13 September 2022 (2022-09-13)<br>entire text, all drawings | 1-10 |
| A | JP 2020-61066 A (FUJITSU LTD) 16 April 2020 (2020-04-16)<br>entire text, all drawings | 1-10 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **31 January 2023** | **07 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/043350**

| Patent document cited in search report | | | Publication date (day/month/year) | Patent family member(s) | | | Publication date (day/month/year) |
|---|---|---|---|---|---|---|---|
| WO | 2018/116921 | A1 | 28 June 2018 | US | 2020/0042883 | A1 | |
| | | | | entire text, all drawings | | | |
| JP | 2022-539791 | A | 13 September 2022 | US | 11132607 | B1 | |
| | | | | entire text, all drawings | | | |
| | | | | EP | 3907655 | A1 | |
| | | | | entire text, all drawings | | | |
| | | | | KR | 10-2021-0157426 | A | |
| | | | | entire text, all drawings | | | |
| JP | 2020-61066 | A | 16 April 2020 | US | 2020/0117991 | A1 | |
| | | | | entire text, all drawings | | | |
| | | | | EP | 3637320 | A1 | |
| | | | | entire text, all drawings | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020533725 A

**Non-patent literature cited in the description**

- A multi-scale conditional deep model for tumor cell ratio counting. **COSATTO, E.** ; **GERARD, K.** ; **GRAF, H. P.** ; **OGURA, M.** ; **KIYUNA, T.** ; **HATANAKA, K. C** ; **HATANAKA, Y.** Medical Imaging 2021: Digital Pathology. SPIE, February 2021, vol. 11603, 31-42